# EUROPEAN PATENT APPLICATION

(11) **EP 4 597 444 A1**
(43) Date of publication of application: **06.08.2025**
(21) Application number: 25155735.1
(22) Date of filing: 04.02.2025
(51) Int. Cl.: G06V 10/26, G06T 7/10, G06T 7/60, G06V 10/46, G06V 10/48, G06V 10/764, G06V 20/69

(54) **NEW APPARATUS FOR PERFORMING SERUM-AGGLUTINATION TESTS**

(30) Priority: 05.02.2024 IT 202400002301
(71) Applicant: Diesse Diagnostica Senese S.p.a., 20157 Milano (IT)
(72) Inventor: NORELLI, Michele, 53035 Monteriggioni (SI) (IT); BERRETTI, Mirko, 53035 Monteriggioni (SI) (IT); MASSARI, Thomas, 53035 Monteriggioni (SI) (IT)
(74) Representative: Botti & Ferrari S.p.A.

(57) **Abstract**

An apparatus for performing serum-agglutination tests is described, the apparatus having a housing area configured to house a plate with a plurality of wells containing a sample to be analyzed, a processing unit adapted to manage the apparatus, and at least one image detector in communication with the processing unit for acquiring images of the plate located in the housing area. The processing unit is configured to control the acquisition of an image containing at least one reaction well, extract detectable metric features of an agglutinate from said image, and, based on said metric features, calculate a numerical index indicative of the positivity degree of the sample in the well, said calculation being performed with reference to a mapping wherein a determined numerical index corresponds to a determined metric feature.

## Description

### Field of application

The present invention refers to an apparatus for performing serum-agglutination tests in a microplate, for example for the identification of infectious diseases. The following description refers to this field of application with the only purpose to simplify its exposition.

### Prior art

As known, serum-agglutination tests are used to show the presence of antibodies against corpuscular antigens, through the agglomeration of antigen/antibody complexes which causes the formation of a precipitate (referred to as "agglutinate" in the art) visible to the naked eye. In other words, the term "agglutination" indicates a phenomenon through which, under specific conditions, bacteria, red blood cells, and other corpuscular elements suspended in a liquid assemble in clumps and precipitate, and it is thus the visible manifestation of the aggregation of antibodies and antigens.

The above-mentioned serum-agglutination tests allow to verify the positivity of a sample and also the semiquantitative determination of antibodies through titration, which is an operation consisting in the use of progressive dilutions of the serum under test and detection of the highest dilution (i.e. lowest concentration) at which the antigen/antibody reaction causes agglutination ("antibody titer").

The agglutination tests are simple to perform and have a wide range of applications to infectious and non-infectious diseases. For example, it is possible to make a diagnosis of enteric fever/typhus fever through bacterial agglutination (in which the antigen - hereinafter referred to as "Ag" - consists for example in a bacterial suspension), or a diagnosis of syphilis through an RPR test (Ag with charcoal) and/or TPHA test (Ag on Red Blood Cells), as well as rheumatology tests (for example, ASO, RF, CRP with Ag on latex), diagnosis of mononucleosis (Ag on latex) and determination of the ABO blood group.

To this day, said methods are still the reference test for the diagnosis of some infectious diseases such as for example salmonellosis, brucellosis, and syphilis, and are often performed manually (for example, by performing doubling dilutions of the serum under test) with a variety of approaches (on a slide, on suitable cardboards, in a tube, in a microplate). Manual tests require a visual interpretation of the data and the results are often obtained after long incubations (lasting hours) of the reaction mixture, wherein the analysis is hardly objective and it is difficult to interpret the result, since the interpretation is highly dependent on the operator's ability and experience.

There are apparatuses capable of performing serum-agglutination tests in an automatic way, for example through the analysis of the images of the microplate containing the samples, although said apparatuses are often not free from flaws, such as for example the difficulty of extracting from the image analysis features that are not the mere positive/negative discrimination, as well as their poor reliability.

The technical problem of the present invention is to provide an apparatus having functional and structural features capable of overcoming the limitations and the drawbacks reported in connection to the prior art, in particular an apparatus that allows to perform serum-agglutination tests in an automatic and reliable way, thereby obtaining quantitative information.

### Summary of the invention

The solution idea underlying the present invention is to make an apparatus capable of performing in an automatic way serum-agglutination tests through automated analysis of images of the microplate containing the reaction wells; said analysis allows to extract significant metric features from the acquired images (such as, for example, the identification of circular crowns and the possible calculation of magnitudes (sizes) such as their perimeter, or the difference of images obtained at a different time to verify the amount of agglutinate produced), thereby obtaining quantitative information regarding the sample under test. In particular, thanks to the above-mentioned metric features, it is possible to provide a percentage index of positivity of the sample under test.

Based on said solution idea, the above-mentioned technical problem is solved by an apparatus for performing serum-agglutination tests, comprising a housing area configured to house a plate with a plurality of wells containing a sample to be analyzed, a processing unit adapted to manage the apparatus, and at least one image detector in communication with the processing unit for acquiring images of the plate located in the housing area, wherein the processing unit is configured to control the acquisition of an image containing at least one reaction well (in particular, to obtain the image of single wells and to process said images, for example starting from the image of the plate and cutting out said wells), extract detectable metric features of an agglutinate from said image, based on said metric features, calculate a numerical index indicative of the positivity degree of the sample in the well, said calculation being performed with reference to a mapping wherein a determined numerical index corresponds to a determined metric feature, and possibly, based on said numerical index, classify the sample contained in the well, providing information on the positivity of said sample through said classification.

More in particular, the invention comprises the following additional and optional features, taken individually or in combination if needed. Said aspects are in particular described, for example, in the dependent claims 2 to 13, individually or in combination with each other.

According to an aspect of the present invention, the processing unit may be configured to perform a tests of the Treponema Pallidum Haemagglutination Assay (TPHA) type.

More in particular, the processing unit may be configured to verify that, when the sample is combined with treponemal antigens attached to avian erythrocytes, an agglutinate characterized by a circular or semicircular shape (for example, having a certain perimeter) is formed in the image of the well, and/or the presence of a circular or semicircular shape within the agglutinated is formed; in general, the processing unit is configured to identify circular crowns.

According to an aspect of the present invention, the processing unit may be configured to identify a circular contour having a size (or magnitude), such as for instance the perimeter, beyond a first pre-fixed threshold in the image; identify a second circular contour inside said circular contour; measure a size, such as for example the perimeter, of said second circular contour and assign a value proportional to said measured quantity to said numerical index. Should the second circular contour not be identified, the processing unit may be configured to identify a single circular contour having a quantity or size, such as for example the perimeter, beyond a certain second threshold (which may be different from the first threshold, for example lower, but also higher than or it may also be equal to the first threshold) and, should said circular contour be identified, assign a numerical index equal to zero, said circular contour being representative of a negative sample. Should no circular contour be identified in the preceding steps, the processing unit may be configured to assign a numerical index equal to one hundred, representative of a positive sample, in particular very positive (and, in this case, an intermediate step with searching of a condition similar to a circle inscribed in a circle may be carried out, but using loosened circularity criteria).

According to an aspect of the present invention, the processing unit may be configured to perform a test of the Widal Wright (WW) and Weil Felix (WF) type.

According to an aspect of the present invention, the processing unit may be configured to verify whether or not there is any agglutinate in the image when the sample is combined with a specific bacterial suspension (for example colored).

More in particular, the processing unit may be configured to acquire a first image at a first time (for example, an initial time) and a second image at a second time (for example, a final time) of the plate (in this case, the images may contain several reaction wells for the various suspensions, said wells being then analyzed individually, for example by cutting out the single wells); subtract the first image and the second image from each other pixel by pixel, thus obtaining a subtraction image; calculate the sum of the pixels different from zero in said subtraction image; calculate the numerical index indicative of the positivity degree of the sample based on said sum.

According to an aspect of the present invention, the processing unit may be configured to preliminarily binarize the acquired image.

According to an aspect of the present invention, the numerical index may correspond to a percentage value of positivity.

According to an aspect of the present invention, the processing unit may be configured to establish the positivity of a sample if said percentage value is above or below or equal to a pre-fixed threshold.

According to an aspect of the present invention, said threshold may be adjustable.

According to an aspect of the present invention, the processing unit may be configured to identify single wells inside the plate by identification of circular contours having a certain radius; identify coordinates of the centers of said circular contours according to a Cartesian reference system; cluster said centers based on their coordinates calculated above.

According to an aspect of the present invention, the processing unit may be configured to acquire and process a plurality of images of a respective plurality of wells, each of said wells corresponding to a specific serial dilution of the sample therein contained, thus producing as output a titration curve in relation to the sample contained in said wells, wherein, for each dilution, the processing unit is configured to perform the classification of the sample. Said titration curve may then be compared with stored known curves.

According to an aspect of the present invention, the processing unit may be programmed to automatically identify, inside the plate, based on the instructions therein stored, a series (array) of said wells with serially diluted sample, and analyze said wells consecutively.

According to an aspect of the present invention, based on an acquired image (for example, a preliminary image, but non necessarily a preliminary image, it may also be the image that is then processed as described above), the processing unit may be configured to verify the actual presence of the plate, to verify that said plate is the plate associated with a set test, and to verify the orientation of the plate, for example relative to a reference system.

The present invention also relates to a method for performing serum-agglutination tests, comprising the steps of:
- controlling the acquisition of an image containing at least one reaction well (in particular, to obtain the image of single wells and to process said images, for example starting from the image of the plate);
- extracting detectable metric features of an agglutinate from said image;
- based on said metric features, calculating a numerical index indicative of the positivity degree of the sample in the well, said calculation being performed with reference to a mapping wherein a determined numerical index corresponds to a determined metric feature; and
- possibly, based on said numerical index, classifying the sample contained in the well, providing information on the positivity of said sample through said classification.

According to an aspect of the present invention, a test of the Treponema Pallidum Haemagglutination Assay (TPHA) type may be performed through the above-mentioned method.

In particular, when the sample is combined with treponemal antigens attached to avian erythrocytes, the step of extracting the metric features may comprise the step of establishing whether an agglutinate characterized by a circular or semicircular shape (for example, having a certain perimeter) is formed in the image of the well, and/or the presence of a circular or semicircular shape within the agglutinate is formed.

According to an aspect of the present invention, a test of the Widal Wright (WW) and Weil Felix (WF) type may be performed through the above-mentioned method.

In particular, when the sample is combined with a specific bacterial suspension (for example, colored), the step of extracting the metric features may comprise the step of establishing whether or not there is any agglutinate in the image.

The features and advantages of the apparatus and the method according to the invention will become apparent from the following description of an embodiment thereof, given by way of non-limiting example with reference to the accompanying drawings.

### Brief description of the figures

In the figures:
- Figure 1 is a view of the apparatus according to the present invention;
- Figure 2 shows an intentionally simplified outline of the apparatus according to the present invention;
- Figures 3A-3D show examples of images of a reaction well for a negative, a weakly positive, a strongly positive and an extremely positive sample in the case of a TPHA test;
- Figures 4A-4D show examples of images of a reaction well for different positivity degrees in the case of WW and WF tests;
- Figure 5 shows an image of a plate portion in which with each well is associated a respective sample;
- Figures 6A-6C show examples of image processing according to the present invention;
- Figure 7 shows an example of definition of percentage of positivity of a sample in a TPHA test;
- Figure 8 shows an example of definition of percentage of positivity of a sample in a WW and WF test; and
- Figures 9A-9C show examples of images of a reaction well for different positivity degrees in the case of an RPR test.

### Detailed description

With reference to those figures, an apparatus for performing serum-agglutination tests according to the present invention is globally and schematically indicated with the reference number 1.

It is worth noting that the figures represent schematic views and are not necessarily drawn to scale, but instead they are drawn so as to emphasize the important features of the invention. Further, in the figures, the different elements are shown in a schematic way, their shape being variable depending on the desired application. It is further worth noting that in the figures identical reference numerals refer to identical elements in shape or function. Finally, particular features described in relation to an embodiment illustrated in a figure are also applicable to other embodiments illustrated in the other figures.

It is also noted that, unless the opposite is expressly indicated, the described process steps may also be inverted if necessary.

The present description shows an apparatus applicable to the diagnosis of infectious diseases, even if the present invention is not limited to this application.

In particular, the procedure detailed below applies to performing TPHA (acronym for "Treponema Pallidum Haemagglutination Assay") tests, wherein specific antibodies in the patient's serum bind to treponemal antigens attached to avian erythrocytes, and to the analysis of bacterial-agglutination reactions for the detection of antibodies against Salmonella, Brucella and Rickettsia, i.e., it is also capable of performing the Widal-Wright (hereinafter simply referred to as WW test) and Weil-Felix (hereinafter simply referred to as WF test) tests. The methodology and the examples described below thus apply to the above-mentioned tests, although other tests in addition to the latter are not excluded.

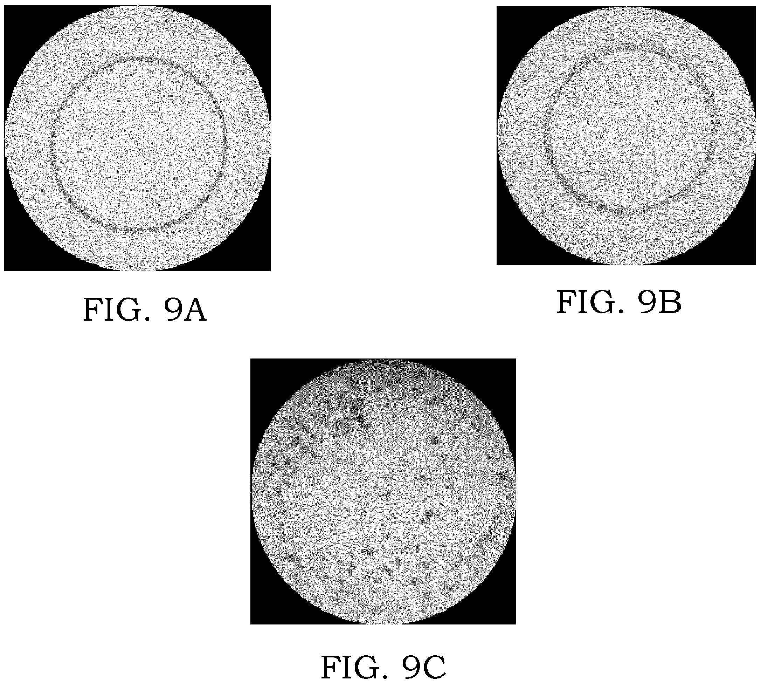

For example, in addition to the above-mentioned tests, it is also possible to perform RPR (acronym for "Rapid Plasma Reagin") tests with charcoal for the analysis of non-treponemal agglutination reactions for searching aspecific antibodies against T. Pallidum, in which there is formation of a ring of the sample (as in Figures 9A-9C), although TPHA and WW-WF tests will be detailed below in the present description.

For the purpose of allowing the execution of the operations described below, the apparatus 1 comprises a processing unit (identified with reference C), including an appropriate memory unit MEM and suitably programmed and designated for managing and automatically controlling the apparatus and for the analysis of the data of measurement. The processing unit C may be, for example, a computerized unit integrated in the apparatus 1. Moreover, it is noted that the processing unit C may be a single unit or may comprise a plurality of local and/or remote units, possibly communicating with each other and each being designated for performing specific operations. The processing unit C is thus capable of controlling the apparatus 1 to obtain the desired functionalities. In any case, the present invention is not, in any way, limited by the architecture used for the processing unit C, which may be in general any suitable computerized unit, comprising one or more unit(s) depending on the needs and/or circumstances. Only by way of example, an electronic board IMX8 may be used as CPU.

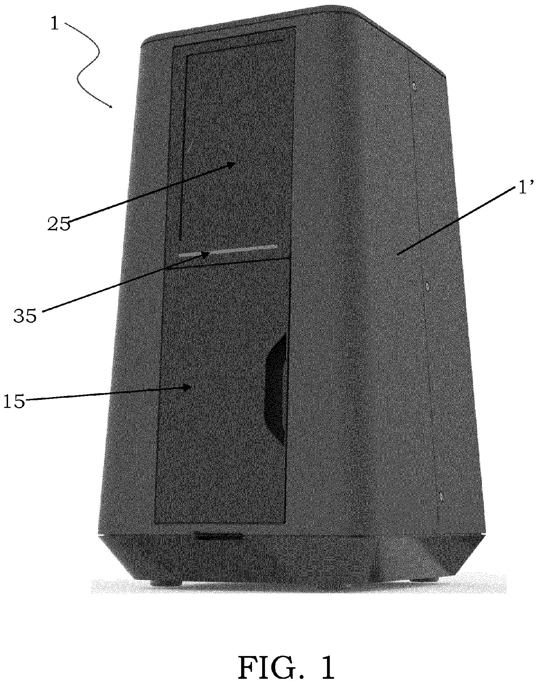

As illustrated in Figure 1, in its most general form, the apparatus 1 comprises a casing (reference 1') containing its main hardware components, but it is not limited to a given aesthetic shape.

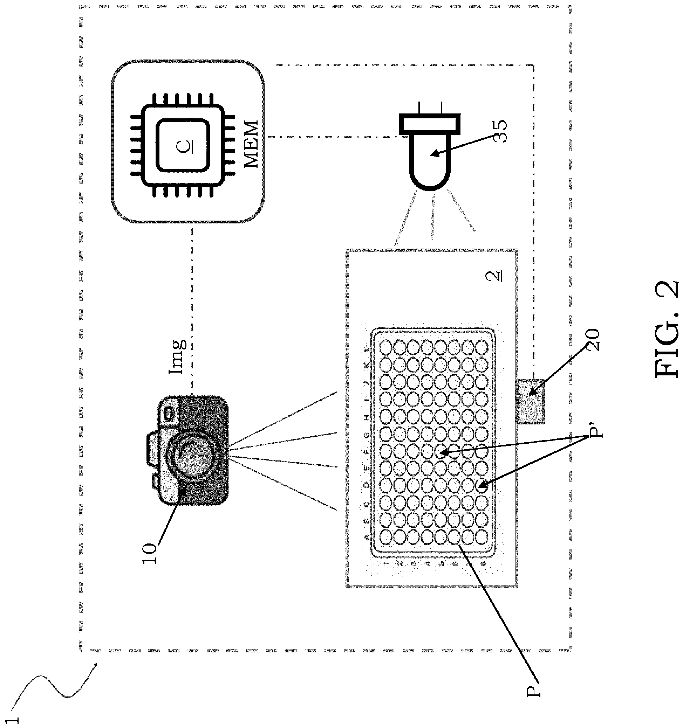

As schematized in Figure 2 (which is an intentionally schematic illustration of the instrument), there is also a housing area (reference number 2) configured to house a plate (or microplate, indicated with the reference P) containing a plurality of reaction wells P' in which there is a sample to be analyzed. By way of example, the plate P may contain ninety-six reaction wells.

The apparatus 1 further comprises a reading unit including at least one image detector (reference 10) in communication with the processing unit C for acquiring images (indicated with the reference Img) of the plate P arranged in the housing area.

In an embodiment, the image detector 10 is a camera having high resolution (for example, from 10 MP to 20 MP) and capable of adjusting the focus, the exposure and other parameters. The camera may also be provided with ad hoc optics capable of minimizing the distortion effect. Moreover, there may be two cameras (each camera framing a respective plate portion, and the overall image may thus be obtained by combining the images of said single cameras), however, the present invention is not limited by said configuration.

The processing unit C is configured to process the images Img acquired by the image detector 10 and to perform, based on said processing (in particular, based on an image-processing algorithm that said processing unit runs), a serum-agglutination test relative to the samples contained in the wells P' of the plate P. The purpose of said processing is to extract significant features from the images, in particular geometrical features, to provide an accurate numerical result.

As indicated above, regardless of the configuration of the processing unit C, the apparatus 1 is suitably configured for the analysis of reactions of treponemal hemagglutination for searching T. pallidum-specific antibodies, i.e., to perform TPHA tests, wherein specific IgG/IgM antibodies in the serum of the patient bind to the treponemal antigens attached to avian erythrocytes, to assess the exposure to syphilis. It is thus a treponemal test to detect the presence of antibodies specific for Treponema antigens in the sample. In this case, U-bottom microtitration plates may be used.

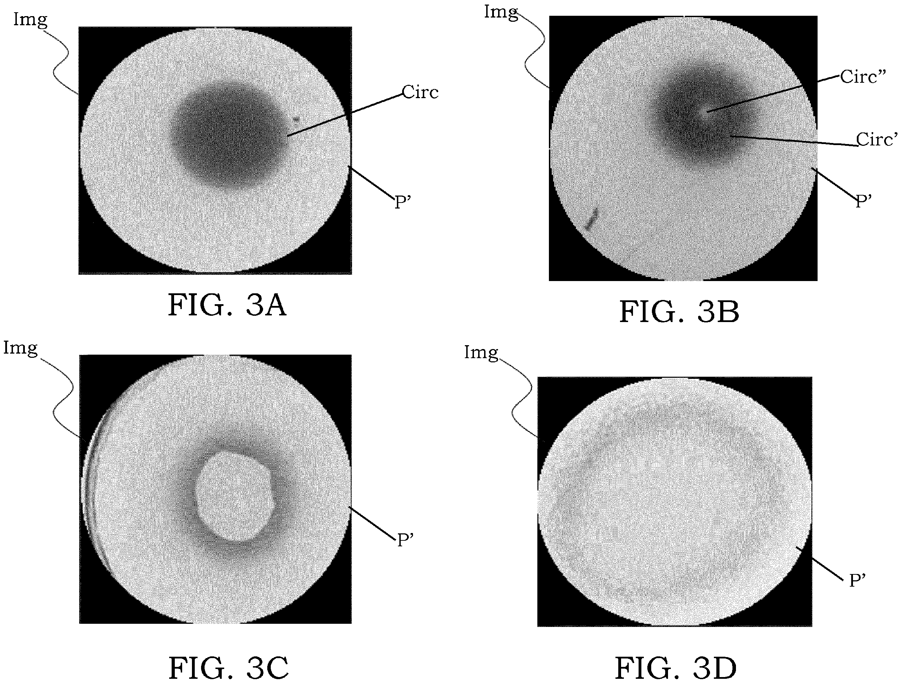

Figures 3A-3D show examples of images obtained and analyzed in a TPHA test, wherein Figure 3A shows a typical negative sample, Figure 3B a weakly positive sample, Figure 3C a strongly positive sample and Figure 3D an extremely positive sample. In this case, the processing unit C is configured to verify that, when the sample is combined with treponemal antigens attached to avian erythrocytes, in the image Img a dispersed agglomerate characterized by a circle having a certain perimeter and/or the presence of a ring/circle within the agglutinate is formed, as will be detailed below. As a matter of fact, in the absence of agglutination (and thus in negative samples), the erythrocytes sediment, thereby forming a compact mass having a substantially circular shape in the center of the well, whereas in the presence of agglutination the erythrocytes do not sediment, thereby forming a layer which is homogeneously spread on the bottom of the well and/or forming a characteristic ring structure. The procedure performed by the processing unit C is thus adapted to identify said features, extracting parameters representative of the agglutinate, such as for example the internal gradient of the structure formed, the size of the possible internal hole, and the outer perimeter, wherein said parameters, when compared with each other, provide information about the positivity of the sample.

Moreover, as mentioned above, the apparatus 1 is capable of analyzing bacterial-agglutination reactions for the detection of specific antibodies against Salmonella, Brucella and Rickettsia, i.e., it is also capable of performing the WW and WF tests. In this case, seven specific bacterial suspensions (for example colored) may be used for the serological diagnosis of infections by Salmonella and Brucella, and thus for performing the WW test for the diagnosis of, for example, typhoid fever and brucellosis (TO - Salmonella Typhi, somatic antigen O, TH - Salmonella Typhi, flagellar antigen H, AO - Salmonella Paratyphi A, somatic antigen O, AH - Salmonella Paratyphi A, flagellar antigen H, BO - Salmonella Paratyphi B, somatic antigen O, BH - Salmonella Paratyphi B, flagellar antigen H, BR - Brucella) together with a positive-control polyvalent serum and with a negative control, and three specific bacterial suspensions (for example colored) are used for the serological diagnosis of infections by Rickettsia (Proteus OX19, OXK, OX2, i.e. for searching specific anti-Proteus antibodies), and thus for performing the WF test, together with a positive-control polyvalent serum.

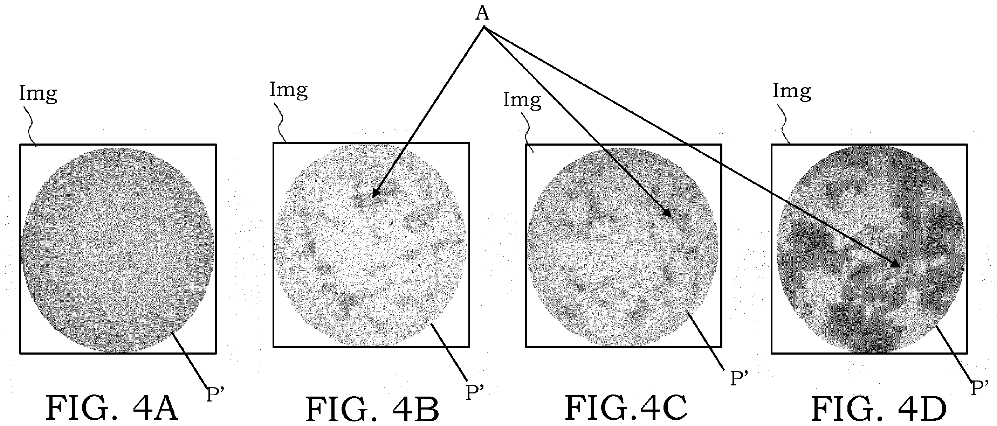

Figures 4A-4D show examples of images obtained and analyzed in WW and WF tests. In this case, the processing unit C is configured to verify whether or not there is any agglutinate (referred to with the reference "A") in the image Img when the sample is combined with a specific bacterial suspension (for example colored), as will be discussed below, wherein the presence of agglutinate A is indicative of a positive sample.

The following description will detail the procedure of image processing for performing TPHA and WW-WF tests.

First of all, in all the tests performed through the instrument, a preliminary procedure of verification of the correct setup of the apparatus 1 is performed. For example, in this preliminary procedure, an image Img of the plate P is acquired and, based on this image Img, it is possible to verify the actual presence of the plate, P, and it is also possible to verify that said plate P is exactly the plate associated with the set test, i.e., if it is the correct plate for said test. As it will be discussed hereinafter, the apparatus 1 is in fact capable of performing different agglutination tests, each characterized by its own specific plate, and thus allows the user to set the desired test, said setting corresponding to the automatic selection, by the processing unit C, of the respective operating instructions, which is the reason why it is important to verify that the plate P inserted is the correct one. In this preliminary procedure, it is further possible to verify the orientation of the plate P according to a pre-fixed reference system.

These preliminary procedures, as well as other processing steps discussed below, may be performed by means of machine-learning techniques.

For what concerns the specific procedure of image processing for performing the test, first of all, the processing unit C cuts out the regions of interest to be processed. In particular, running the algorithm provides that circular contours within a certain radius range (the latter being compatible with the measurement of the known radius of the well P') are identified in the plate P. For example, the identification of the circular contours may be based on the HoughCircle class. Once the coordinates of the centers of said circles according to a Cartesian reference system have been identified (i.e., once their abscissa and ordinate have been defined), the centers are clustered based on their coordinates; in particular, the candidate centers of the circles are clustered based on their abscissa and ordinate values, and clusters with a sufficiently high numerousness (i.e., the clusters having the same ordinate or the same abscissa) are plausible candidates for the position of a column or a row of wells. Possible positions whose ordinate or abscissa have not been found are reconstructed based on the values of the expected pitch between the wells P' and on the relative distances of the clusters' centers actually found.

For the sake of easier description, the images of the single wells P' will be always indicated with the reference "Img" used for the image of the plate P.

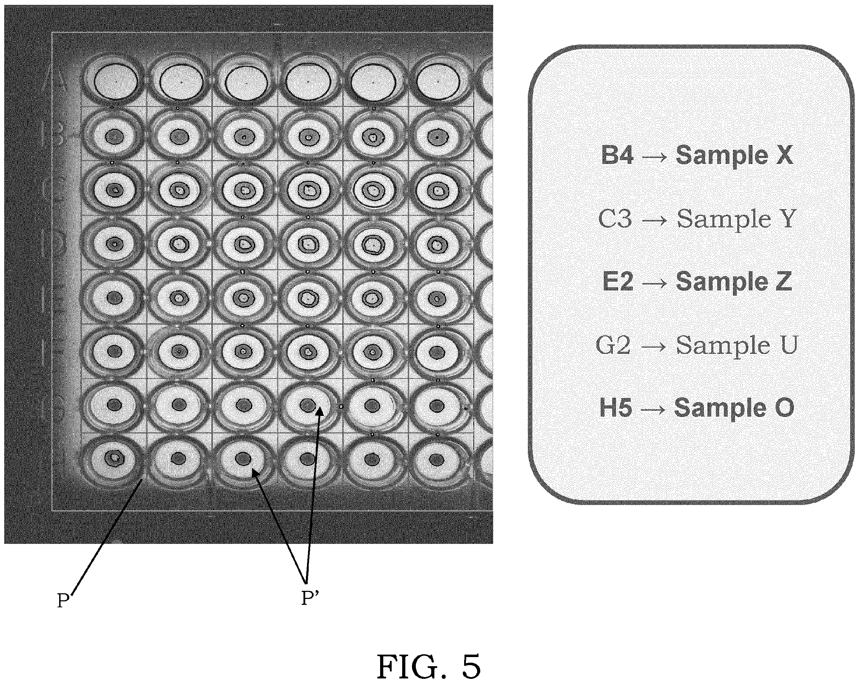

It is also noted that, since the plate P may contain a high number of wells (for example, ninety-six), the processing unit C is programmed to associate with the identified single wells P' respective samples whose features (for example, the data identifying the patient) are defined in its memory unit MEM. This is shown in Figure 5 (showing the image of a portion of the plate P), in which with each position of the well in a defined grid is associated the respective sample.

The above-mentioned procedures are a common aspect of the apparatus 1 and are performed both for the TPHA test and for the WW and WF tests.

Suitably, the processing unit C is further configured to preliminarily binarize the acquired image Img, in particular the image of the single well P', prior to extracting the parameters of interest, so that said parameters are obtained from the binarized image Img.

For example, the image Img of the single well P° is first translated into gray scale and subsequently binarized.

More in particular, the processing unit C produces two different binary images through two adaptive approaches of threshold extraction: a first binarized image is produced through Otsu binarization (said image being indicated as bin1 below) and a second binarized image through the K-means method (said image being indicated as bin2 below).

The binarization allows to have a good contrast and makes the identification of the desired features easier (for example, it facilitates searching a central hole, as will be illustrated below).

In this way, for each well P', a series of binarized images is defined, on which images some quantities characterizing the image Img are then extracted, as described below.

In particular, advantageously according to the present invention, as mentioned above, the processing unit C is configured to extract a plurality of features to be used to classify the sample. More in particular, metric features of the agglutinate are extracted from the image Img and, based on said metric features, a numerical index indicative of the positivity degree of the sample in the well P' is calculated. The calculation of the numerical index of positivity is performed with reference to a mapping wherein a determined numerical index corresponds to a determined metric feature. The obtained metric features thus provide information about the morphology of the agglutinate formed following the reaction in the well P'.

The above-mentioned mapping of the positivity of the sample is thus defined through a linear extrapolation/combination of the obtained results, since the obtained metrics are easily scalable from zero to one hundred, so as to obtain an optimal quantification.

The numerical index calculated and assigned to each sample is thus an output corresponding to a percentage value of the positivity of the sample and allows to provide a quantitative result and not just a qualitative one.

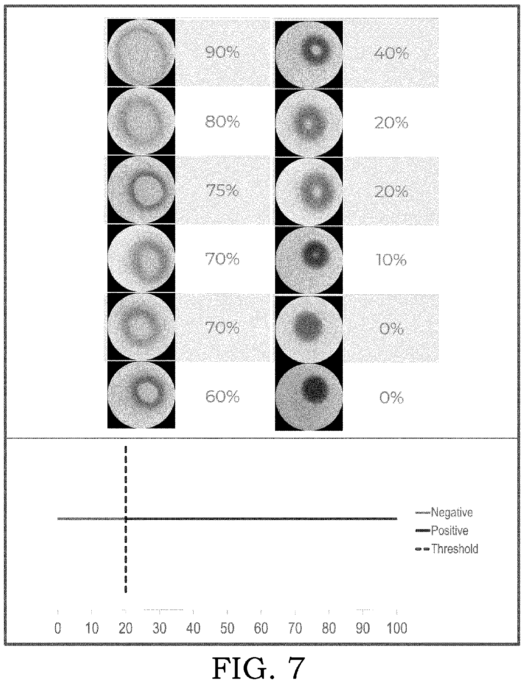

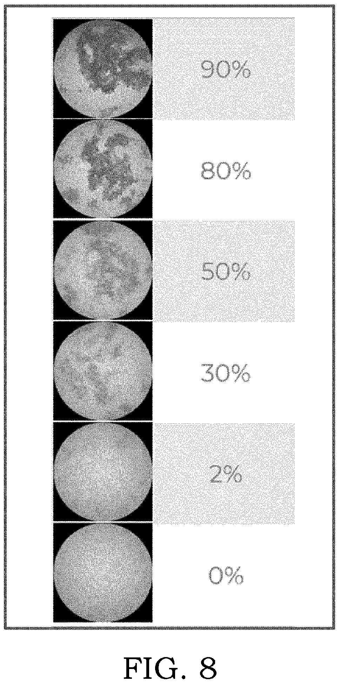

In this way, the processing unit C is configured to define, based on the combination of the metric features extracted from the image Img, a percentage of positivity of the sample (for example, the maximum percentage corresponds to the maximum positivity degree, whereas the minimum percentage corresponds to a single circle defined for the TPHA tests and to the absence of agglutinate for the WW and WF tests, thus to a negative sample, as illustrated in Figures 7 and 8). It is thus possible to set a positivity threshold, i.e., it is possible to define a percentage value beyond which the sample is considered positive, thereby obtaining an even finer analysis. In other words, the numerical index defined as above corresponds to a percentage value, and the processing unit C is configured to establish the positivity of a sample if said percentage value is above or below a pre-fixed threshold. Suitably, the threshold is adjustable.

In this way, the information obtained goes beyond the mere positive/negative discrimination, since it is possible to detect all the possible nuances of positivity, obtaining a numerical value for which thresholds can be defined, for example based on known results.

Based on the calculated numerical index, it is then possible to classify the sample contained in the well P', providing information on the positivity of said sample through said classification.

In the case of performing a TPHA test, as seen above, the processing unit C is configured to verify that, when the sample is combined with treponemal antigens attached to avian erythrocytes, in the image Img of the well P' an agglomerate characterized by a circle having a certain perimeter and/or the presence of a ring defined in the agglutinate is formed.

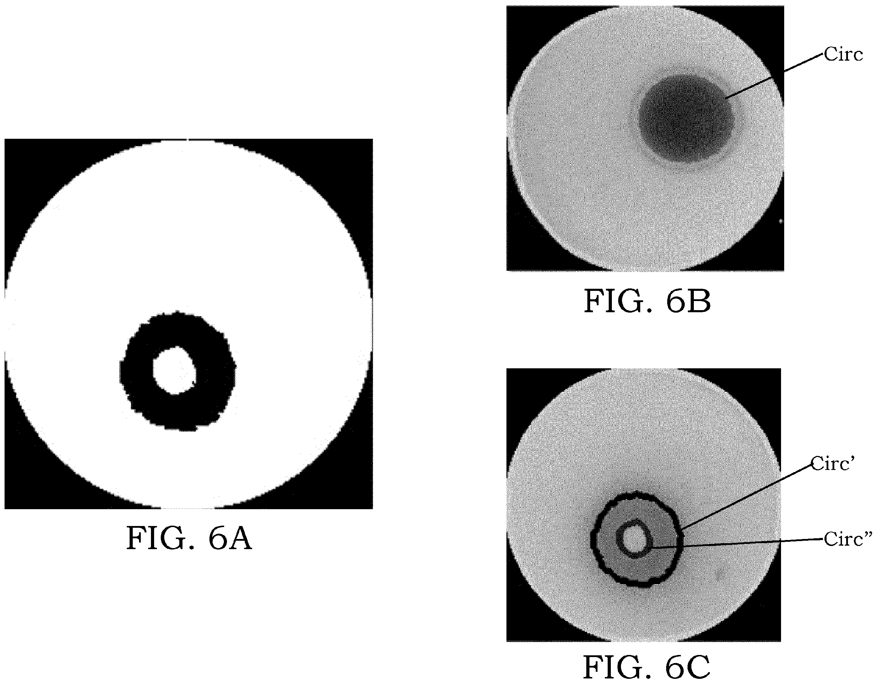

In particular, both a qualitative determination of positivity/negativity and, advantageously, also a quantitative determination of the positivity level in a wide range are performed. At the biological level, indeed, a proportionality between the positivity level of a given sample and the radius of the perimeter within the agglutinate is found (in this regard, see Figure 6C); the processing unit C is thus capable of extracting and measuring said quantity.

More in particular, in the TPHA test, for each image Img of the single wells P', the processing unit C produces the above-mentioned numerical index in the following way. First of all, all the closed contours above a certain length (for example, beyond a certain perimeter/diameter) are searched. Once said contours have been found, it is searched if there is a circular contour (with a perimeter beyond a certain first threshold) containing a second circular contour which is concentric therewith (except for a tolerance), as in Figures 3B and 6C. If said configuration exists, the returned index is a quantity proportional to the inner perimeter of the circle thereby found (or to a quantity equivalent thereto, such as for example the diameter). Said numerical index is thus representative of a positive sample, as previously described. The circular contours are identified by using algorithms for image processing for searching circular shapes, such as, for example, algorithms based on the Hough transform.

If the above-described configuration is not found, it is then verified if there is a single circular contour (having a perimeter beyond a second threshold, generally smaller than the first threshold relative to the circle of the positive samples, but not necessarily, it may also be equal to or greater than it), representative of a negative sample (as in Figure 3A), and, in this case, if said circular contour is found, a numerical index of zero, which is indicative of a negative sample, is assigned.

If none of the above conditions is found, it is verified whether there is a condition similar to the first one (i.e., a circular contour above a first threshold, possibly containing a second circular contour which is concentric therewith) but using loosened circularity criteria, as in Figure 3C. Said configuration is a sign of a very positive configuration (to which, for example, a numerical index of 100 corresponds).

Should no contour having a perimeter above a threshold be found, the configuration is most likely extremely positive (to which, as in the previous case, a numerical index of 100 corresponds).

Generally, it is thus a both qualitative and quantitative analysis, with combined search for a circle-point: a point or a full totally black circle in the center of the well corresponds to a negative sample, whereas a circle with a variable diameter, possibly containing a circle within it, is a sign of a positive sample, and, the larger the diameter, the more positive is the sample.

In other words, summarizing briefly, the processing unit C is configured to:
- identify a circular contour (reference Circ') having a size or magnitude(for example, the perimeter) beyond a first pre-fixed threshold in the image Img;
- identify a second circular contour (reference Circ") inside said circular contour;
- measure a size, such as for example the perimeter, of said second circular contour (Circ") and assign a value proportional to said measured quantity to said numerical index;
- should the second circular contour (Circ") not be identified, identify a single circular contour (generally indicated as Circ) having a quantity or size (for example, the perimeter) beyond a certain second threshold (which may be, but not necessarily, different from the first threshold, - in particular, but not necessarily, lower), and, should said circular contour Circ be identified, assign a numerical index equal to zero, said circular contour being representative of a negative sample; and
- should no circular contour beyond the first or the second threshold be identified in the preceding steps, assign a numerical index equal to one hundred, representative of a positive sample.

This analysis is performed for each of the two binarized images bin1 and bin2, and there are thus two indexes to be analyzed (one for each previously defined threshold with which said binarized images bin 1 and bin2 are obtained. The end determination result of the test is obtained through the following logic: if the numerical index obtained from image bin1 is strongly positive or if it is between a set threshold and 100, then the final index is determined with the index provided by image bin2, which is supposed to be more sensitive to strongly positive samples; in all the other cases, the analysis output is provided by the numerical index obtained by image bin 1.

In the case of Widal Wright (WW) and Weil Felix (WF) tests, as seen above, the processing unit C is configured to verify whether or not there is any agglutinate in the image Img when the sample is combined with a specific bacterial suspension (in particular colored).

Referring again to Figures 4A-4D, images of wells with increasing positivity are shown from left to right, and the calculation procedure carried out by the processing unit C is capable of measuring the positivity in a quantitative way, by accurately measuring the darker area of the image that constitutes the agglutinate.

More in particular, for each well, the processing unit C processes two images photographed at the beginning of the reaction (time T0) and at the end of the reaction (time TF), and possibly also at least one intermediate image at an intermediate time (although this is not strictly necessary). Subsequently, each image is transformed into gray scale and subtraction of the images pixel by pixel is performed, thereby obtaining a subtraction image (in particular, the final image and the image at the initial time are subtracted, but intermediate images can possibly also be used). The sum of the pixels different from zero is then calculated in said subtraction image. This sum is directly proportional to the amount of agglutinate formed and to its intensity, and thus to the positivity of the sample, thereby providing the numerical index corresponding to the percentage of positivity of said sample. A quantitative result proportional to the stained surface in the well P' is thus obtained.

Thus, also in this case, both a qualitative analysis (determination of positive negative) and a quantitative analysis are obtained, wherein the processing unit C identifies agglutinate formation in the image; in fact, at time T0 the wells P' are totally colored and subsequently, at time TF, in the presence of agglutinate, the wells lose the homogeneous color and darker areas appear. The more the darker areas are, the higher the positivity of the sample is.

It is noted that, differently from the TPHA test, in this case the above-mentioned difference image is calculated, since in the WW and WF tests there are no strong features (circles or circular crowns) to be identified to distinguish a positive sample from a negative sample, and, moreover, possible shades due to the depth of the well P' might reduce the ability of measuring the agglutinate near the edge; finally, the color taken on by the agglutinate might not have a sufficiently wide contrast to always allow its identification through an adaptive threshold on the single image.

In other words, summing up, the processing unit C is configured to acquire a first image at a first (e.g., initial) time and a second image at a second (e.g., final) time;
- subtract the first image and the second image from each other pixel by pixel, thus obtaining a subtraction image:
- calculate the sum of the pixels different from zero (dark pixels) in said subtraction image; and
- calculate the numerical index indicative of the positivity degree of the sample based on said sum.

Thus, in all the above-mentioned TPHA, WW and WF tests, a numerical result and a very accurate classification are obtained, and this is possible thanks to the metric features extracted from the image Img, with a high analysis resolution.

In an advantageous embodiment of the present invention, which is in common for all the types of tests performed by the apparatus 1, a plurality of images of a respective plurality of wells (for example, wells that are adjacent in the microplate) is acquired, each of said wells corresponding to a specific serial dilution of the sample therein contained; thereby, it is possible to produce as output a titration curve in relation to the sample contained in said wells P, in which, for each dilution, a classification of the sample is performed. Essentially, in these wells P', the same amount of antigen is used with progressive dilutions of the serum under test, and the processing unit C is capable of detecting which is the highest dilution at which the antigen/antibody reaction causes agglutination (thereby providing the so-called "antibody titer") .

Combining this fact with the possibility of obtaining a percentage value of positivity of the sample, a quantitative result is obtained in a single well, thus allowing to perform a smaller number of dilutions of the sample (thereby saving space in the microplate), thanks to the fact that quantitative features can already be extracted from the single wells.

In the above-mentioned quantitative analysis, the result may thus be expressed in dilutions, and the processing unit C may compare the obtained result with stored titration curves to assign a titer to the sample.

Suitably, the processing unit C is programmed to automatically identify, inside the plate P and based on the instructions therein stored, the series of said wells P' with serial dilution and to analyze said wells in a consecutive way, i.e., it is capable to analyze, by means of prearranged instructions, arrays of wells with serial dilutions.

Generally, the above-described processing procedure is particularly advantageous since it allows to extract deterministic features from the image, with higher precision on the determination of the thresholds to classify the samples (the threshold may be set at a certain percentage value), and thus with a higher reliability of the measurement and also the possibility to obtain a semiquantitative analysis.

The operation of the specific program of processing the images Img acquired through the apparatus 1 for performing the TPHA and WW-WF tests was described above. As regards the general operation of the apparatus 1, instead, the measurement cycle comprises various steps, and the programming of a cycle may occur both for a single test and for diagnostic profiles, which is particularly useful in the case of bacterial-agglutination tests in which a single serum is tested with different bacterial suspensions. The reagents and the samples are dispensed manually into the plate P following precise indications and, once inserted in the housing area 2 of the instrument, said plate P is processed as follows:
- rapid shaking to mix the sample with the reagent;
- image acquisition at time zero;
- incubation under rotation at constant speed for the time predefined by the specific method (the reaction times are preset and depend on the specific test);

- image acquisition;
- image processing (for example, in the way described above); and
- reading and printing the test results.

The following paragraphs regard details of the mechanical structure of apparatus 1.

As seen above, there is the casing 1 containing all the main components of the apparatus 1, among which the above-mentioned housing area 2. In connection with the latter, it has the function of receiving and holding the plate P prepared by the operator steady inside the instrument, and it acts as the direct interface between the machine and the element to be analyzed. The housing area 2 is slightly flexible to enable to insert easily the microplate inside it, but it is also sufficiently rigid to be hold steady during the steps of shaking and rotation. The housing area 2 is with BioLab 96 well microplates (for which, for example, the above-mentioned method of cutting out the well P' in the image Img was used), and also other types of microplates, without limitation of the scope of the present invention.

In an embodiment of the present invention, the apparatus 1 comprises a door (reference 15) which acts as a closing element of the housing area 2 and which is configured to pass from an open configuration, in which it is possible for the operator to access the housing area 2 to be able to insert or remove the plate P, to a closed configuration, in which it is not possible to access said housing area 2, and vice versa. For security reasons, the door 15 may be provided with a solenoid configured to allow a magnetic closure thereof during the test. The closure of the door 15 may be managed by means of designated software commands inputted through GUI. In an embodiment, there is also a sensor on the door 15 to allow the control of the correct closure thereof, for example a microswitch.

The images Img acquired through the apparatus 1 are saved on site in the memory unit MEM together with the analysis results and are thus available for possible visual checks; the data of measurement, including the instrument's settings, are contained in a designated database.

The apparatus 1 may also connect in a bidirectional way with the Laboratory Information System (LIS), for a complete traceability and security of the analytical data.

As regards the orbital shaking of the microplate, the apparatus 1 further comprises a shaker (reference 20) which is adjustable in speed through appropriate software commands. The shaker 20 thus moves the microplate (for example, it moves said housing area 2 or a support therein) and, in an embodiment, it comprises a step motor which, using helicoidal gears, allows to perform two types of movements for carrying out the steps of analysis, namely, a slow rotation and a fast rotation.

Moreover, the apparatus 1 comprises a display (reference 25), for example a touch-screen display, which may be tilted to allow an easier interaction with the operator. In addition to the operating settings, on the display 25 it is possible to display the acquired images and to confirm the positivity of a sample.

There is also a reader for the identification of the barcode of the samples and of the reagents, and an internal temperature sensor for measuring the inner temperature for the apparatus 1 during the analysis cycle may also be provided.

Moreover, the apparatus 1 comprises a lighting system (reference 30) to illuminate the working area. In particular, there is a first internal lighting system arranged above the plate P which allows to check the lot and the orientation of said plate P with the door 15 in closed configuration. Moreover, under the housing of the microplate, there is a second lighting system (for example, a LED lighting board) for backlighting the wells P'.

Furthermore, on the casing 1', there is a luminous indicator (reference 35, for example an RGB LED) which allows to indicate to the user the current working status of the apparatus 1.

In the light of the above, it is clear that the present invention also refers to a related method for performing serum-agglutination tests, in particular for performing TPHA, WW and WF tests.

In conclusion, summing up, the present invention allows to overcome the technical problem brilliantly, providing the above apparatus and solving all the drawbacks of the prior art, wherein, wherein, when performing a TPHA, WW or WF test, numerous features of the image are extracted through computer-vision techniques, thereby possibly allowing to also provide a semiquantitative result and an antibody titer.

It is thus possible to perform serum-agglutination tests in microplate in an automatic and efficient way, obtaining up to ninety-six results in 15/25 minutes and providing a semiquantitative output in a single well, with archiving of results and images and a complete traceability of the results.

The apparatus 1 is very compact and is capable of carrying out the steps of mixing, incubation and reading in an automatic and efficient way, and the operator is only required to prepare the microplate.

Reading and objective interpretation of the reactions are obtained thanks to the innovative image-processing program described above. Suitably, the image processing is extremely detailed since it allows to extract a very precise metric of the sample, with a higher reliability of the test results. Thanks to the obtained metric features, not only a qualitative discrimination between a positive or negative sample is obtained, but also an actual quantitative analysis, thereby obtaining a percentage of positivity, i.e. a numerical output which is very useful in the analysis.

The quantitative results, expressed through the above-defined numerical index that is related to the measured metric, are thus obtained from the pattern of agglutination in the single wells. This also allows to reduce the number of serial dilutions to extract the titer of the sample, since it is already possible to map the positivity scale with single wells.

Advantegously, the processing unit C of the present invention is configured to calculate the numerical index of the positivity percentage with reference to a mapping in which to a give metric features corresponds a given percentage value. The metric features yield information on the morphology of the agglutinate after the reaction.

Further, the processing unit C is programmed to execute innovative procedures to perform a test of the Treponema Pallidum Haemagglutination Assay (TPHA) type, and/or to perform a test of the Widal Wright (WW) and Weil Felix (WF) type.

Obviously, a person skilled in the art, in order to meet particular needs and specifications, may carry out several changes and modifications to the apparatus described above, all included in the protection scope of the invention as defined by the following claims.

## Claims

1. An apparatus (1) for performing serum-agglutination tests, comprising:
- a housing area (2) configured to house a plate (P) with a plurality of wells (P') containing a sample to be analyzed;
- a processing unit (C) adapted to manage the apparatus (1); and
- at least one image detector (10) in communication with the processing unit (C) for acquiring images (Img) of the plate (P) located in the housing area (2),
wherein the processing unit (C) is configured to:
- control the acquisition of an image (Img) containing at least one reaction well (P');
- extract metric features detectable in an agglutinate from said image (Img); and
- based on said metric features, calculate a numerical index indicative of the positivity degree of the sample in the well (P'), said calculation being performed with reference to a mapping wherein a determined numerical index corresponds to a determined metric feature.

2. The apparatus (1) according to claim 1, wherein the processing unit (C) is configured to perform a test of the Treponema Pallidum Haemagglutination Assay (TPHA) type.

3. The apparatus (1) according to claim 2, wherein the processing unit (C) is configured to verify that, when the sample is combined with treponemal antigens attached to avian erythrocytes, an agglutinate **characterized by** a circular or semicircular shape is formed in the image (Img) of the well (P'), and/or the presence of a circular or semicircular shape within said agglutinated is formed.

4. The apparatus (1) according to claim 2 or 3, wherein the processing unit (C) is configured to:
- identify a circular contour (Circ') having a size, such as for instance the perimeter, beyond a first pre-fixed threshold in the image (Img);
- identify a second circular contour (Circ") inside said circular contour (Circ');
- measure a size, such as the perimeter, of said second circular contour (Circ") and assign a value proportional to said measured quantity to said numerical index;
- should the second circular contour (Circ') not be identified, identify a single circular contour (Circ) having a size, such as for instance the perimeter, beyond a certain second threshold and, should said circular contour (Circ) be identified, assign a numerical index equal to zero, said circular contour being representative of a negative sample; and
- should no circular contour be identified as defined by the preceding steps, assign a numerical index equal to one hundred, representative of a positive sample.

5. The apparatus (1) according to claim 1, wherein the processing unit (C) is configured to perform a test of the Widal Wright (WW) and Weil Felix (WF) type.

6. The apparatus (1) according to claim 5, wherein the processing unit (C) is configured to verify whether or not there is any agglutinate in the image (Img) when the sample is combined with a specific bacterial suspension.

7. The apparatus (1) according to claim 5 or 6, wherein the processing unit (C) is configured to acquire a first image at a first time and a second image at a second time;
- subtract the first image and the second image from each other pixel by pixel, thus obtaining a subtraction image:
- calculate the sum of the pixels different from zero in said subtraction image; and
- calculate the numerical index indicative of the positivity degree of the sample based on said sum.

8. The apparatus (1) according to any one of the preceding claims, wherein the processing unit (C) is configured to preliminarily binarize the acquired image (Img).

9. The apparatus (1) according to any one of the preceding claims, wherein the numerical index corresponds to a percentage value, and wherein the processing unit (C) is configured to establish the positivity of a sample if said percentage value is above or below or equal to a pre-fixed threshold.

10. The apparatus (1) according to claim 9, wherein said threshold is adjustable.

11. The apparatus (1) according to any one of the preceding claims, wherein the processing unit (C) is configured to:
- identify single wells (P') inside the plate (P) by identification of circular contours having a certain radius;
- identify coordinates of the centers of said circular contours according to a Cartesian reference system; and
- cluster said centers based on said coordinates.

12. The apparatus (1) according to any one of the preceding claims, wherein the processing unit (C) is configured to acquire and process a plurality of images (Img) of a respective plurality of wells (P'), each of said wells corresponding to a specific serial dilution of the sample therein contained, thus producing as output a titration curve in relation to the sample contained in said wells (P), wherein, for each dilution, the processing unit (C) is configured to perform the classification of the sample, and wherein said processing unit (C) is programmed to automatically identify, inside the plate (P) and based on the instructions therein stored, a series of said wells (P') with serially diluted sample and analyze said wells consecutively.

13. The apparatus (1) according to any one of the preceding claims, wherein, based on an acquired image, the processing unit (C) is configured to:
- verify the actual presence of the plate (P);
- verify that said plate (P) is the plate associated with a set test; and
- verify the orientation of the plate (P).

14. A method for performing serum agglutination tests, comprising the steps of:
- controlling the acquisition of an image (Img) containing at least one reaction well (P');
- extracting detectable metric features of an agglutinate from said image (Img); and
- based on said metric features, calculating a numerical index indicative of the positivity degree of the sample in the well (P'), said calculation being performed with reference to a mapping wherein a determined numerical index corresponds to a determined metric feature.

15. The method according to claim 14, wherein a test of the Treponema Pallidum Haemagglutination Assay (TPHA) type is performed, and wherein, when the sample is combined with treponemal antigens attached to avian erythrocytes, the step of extracting the metric features comprises the step of establishing whether an agglutinate **characterized by** a circular or semicircular shape is formed in the image (Img) of the well (P'), and/or the presence of a circular or semicircular shape within the agglutinate is formed; and/or
wherein a test of the Widal Wright (WW) and Weil Felix (WF) type is performed, and wherein, when the sample is combined with a specific bacterial suspension, the step of extracting the metric features comprises the step of establishing whether or not there is any agglutinate (A) in the image (Img).
